# EUROPÄISCHE PATENTSCHRIFT

(11) **EP 0 988 060 B1**
(45) Veröffentlichungstag und Bekanntmachung des Hinweises auf die Patenterteilung: **13.06.2007**
(21) Anmeldenummer: 98929212.3
(22) Anmeldetag: 02.04.1998
(51) Int. Cl.: A61K 49/00

(54) **SÄURELABILE UND ENZYMATISCH SPALTBARE FARBSTOFFKONSTRUKTE ZUR DIAGNOSTIK MIT NAHINFRAROTLICHT UND ZUR THERAPIE**
ACID-LABILE AND ENZYMATICALLY DIVISIBLE DYE COMPOUNDS FOR DIAGNOSIS WITH NEAR INFRARED LIGHT AND FOR THERAPY
COLORANTS DE SYNTHESE LABILES AUX ACIDES ET CLIVABLES PAR VOIE ENZYMATIQUE POUR LE DIAGNOSTIC AVEC DE LA LUMIERE DANS LE PROCHE INFRAROUGE ET POUR L'USAGE THERAPEUTIQUE

(30) Priorität: 23.04.1997 DE 19717904
(43) Veröffentlichungstag der Anmeldung: 29.03.2000
(73) Patentinhaber: Institut für Diagnostikforschung GmbH an der Freien Universität Berlin, 13353 Berlin (DE)
(72) Erfinder: LICHA, Kai, D-14169 Berlin (DE); RIEFKE, Björn, D-13595 Berlin (DE); SEMMLER, Wolfhard, D-13467 Berlin (DE); WRASIDLO, Wolfgang, D-14193 Berlin (DE)
(74) Vertreter: Seuss, Thomas
(86) Internationale Anmeldenummer: PCT/DE1998/001001
(87) Internationale Veröffentlichungsnummer: WO 1998/047538

(56) Entgegenhaltungen:
- EP-A- 0 175 617
- EP-A- 0 476 408
- EP-A- 0 800 831
- WO-A-96/17628
- WO-A-97/13490
- WO-A-97/13810
- WO-A-98/22146
- US-A- 5 569 587
- PATENT ABSTRACTS OF JAPAN vol. 095, no. 009, 31. Oktober 1995 & JP 07 145148 A (BIO SENSOR KENKYUSHO:KK), 6. Juni 1995
- LICHA, KAI ET AL: "Synthesis and characterization of cyanine dye - poly(ethylene glycol) conjugates as contrast agents for in vivo fluorescence imaging" PROC. SPIE-INT. SOC. OPT. ENG., 1998, 3196, 98-102, XP002079471
- LICHA, K. ET AL: "Synthesis and characterization of cyanine dyes as contrast agents for near-infrared imaging" PROC. SPIE-INT. SOC. OPT. ENG., 1996, 2927, 192-198, XP002079648

## Beschreibung

Die Erfindung betrifft enzymatisch spaltbare Verbindungen zur In-vivo- und In-vitro-Diagnostik mittels Nahinfrarot-Strahlung (NIR-Strahlung), die Verwendung dieser Verbindungen als optische Diagnostika und Therapeutika und diese Verbindungen enthaltende diagnostische Mittel.

Die Nahinfrarotbildgebung ist ein nicht invasives diagnostisches Verfahren, bei dem die hohe Durchlässigkeit biologischen Gewebes für Licht der Wellenlänge 650-1000 nm ausgenutzt wird. Im Gegensatz zu Licht des ultravioletten und sichtbaren Spektralbereichs, das nur in die obersten Millimeter des Gewebes eindringen kann, werden bei Verwendung von Nahinfrarotlicht Eindringtiefen in Gewebe von bis zu mehreren Zentimetern erzielt. Die Ursachen für die prinzipiell geringe Eindringtiefe von Licht sind die Absorption körpereigener Farbstoffe, hauptsächlich des Hämoglobins und des Wassers, die jedoch im Spektralbereich des Nahinfrarotlichtes zwischen 650 und 1000 nm minimale Werte aufweisen. Dieser spektrale Bereich der größten optischen Gewebetransparenz wird daher auch diagnostisches/therapeutisches Fenster genannt (Boulnois, J., Lasers Med Sci 1986, 1:47-66).
Dem Diagnostiker steht hiermit neben den modernen bildgebenden Verfahren, wie Röntgen, Magnetresonanztomographie oder Ultraschalldiagnostik, ein weiteres Verfahren zur bildlichen Gewebedarstellung zur Verfügung (Haller, E.B., Time-resolved transillumination and optical tomography. J Biomed Optics 1996, 1:7-17).

Die Verwendung von NIR-Strahlung zur ortsabhängigen Aufzeichnung von Blutfluß und Oxygenierungsgrad im Gehirn von Säuglingen durch die Detektion der Absorption von Hämoglobin/Deoxyhämoglobin ist ein seit Jahren bekanntes und angewandtes Verfahren (Jöbsis, F.F., Science 1977, 198:1264-67; Chance, B., Leigh, J.S., Miyake, H. et al., Proc Natl Acad Sci USA 1988, 85:4971-75; Benaron D.A. et al., Science 1993, 33: 369A.).

Das wesentliche Problem bei der Nutzung von nahinfraroter Strahlung ist die starke Streuung des Lichtes, so daß selbst bei unterschiedlichen photophysikalischen Eigenschaften von einem scharf begrenzten Objekt und seiner Umgebung sich dieses Objekt nur unscharf abzeichnet. Das Problem nimmt mit wachsender Entfernung des Objektes von der Oberfläche zu und kann als hauptsächlicher limitierender Faktor sowohl bei der Transillumination als auch bei der Detektion von Fluoreszenzstrahlung angesehen werden. Deshalb können Farbstoffe als Kontrastmittel, die die optischen Eigenschaften der Gewebe prägen und zu einer erhöhten Absorption und Fluoreszenz der zu detektierenden Gewebe führen, auch bei geringer Ortsauflösung eine eindeutige Detektion ermöglichen. Dabei kann das Absorptionsverhalten solcher Farbstoffverbindungen als bildgebende Information ausgenutzt werden. Besitzen die Farbstoffe darüberhinaus die Eigenschaft, die absorbierte Energie als Fluoreszenzstrahlung zu emittieren, so kann diese ebenfalls als bildgebende Information genutzt werden. Hierbei wird die gegenüber der Anregungsstrahlung rotverschobene Fluoreszenzstrahlung gesondert detektiert. Der Vorteil besteht u. a. darin, daß das Gewebe selbst im NIR-Bereich eine äußerst geringe Eigenfluoreszenz aufweist und somit der Untergrund minimal ist. (S. Folli et al., Cancer Research 54, 2643-9 (1994); B. Ballou et al., Cancer Immunol. Immunother. 41, 257-63 (1995); X. Li et al., SPIE Vol. 2389, 789-98 (1995)).

In der Fluoreszenzdiagnostik ist die Voraussetzung dafür eine ausreichende, möglichst hohe Differenz in der Fluoreszenzemission zwischen zu detektierendem und umliegendem Gewebe. Dies kann prinzipiell durch eine Differenz in der Konzentration des Fluoreszenzfarbstoffes zu einem bestimmten Zeitpunkt nach Substanzapplikation erreicht werden. Insbesondere für die Diagnostik in tieferen Gewebeschichten ist diese Differenz bei der Verwendung von Substanzen mit unspezifischem Anreicherungsverhalten oft nicht ausreichend.

Der Erfindung liegt daher die Aufgabe zugrunde, neue Verbindungen zur Verfügung zu stellen, welche die Nachteile des Standes der Technik überwinden.

Die Aufgabe wird erfindungsgemäß durch Verbindungen der allgemeinen Formel (I)

(F-L)ₘ-A (I)

gelöst, worin
- F: für ein Farbstoffmolekül mit mindestens einem Absorptionsmaximum zwischen 600 und 1200 nm steht,
- L: für eine Linkerstruktur, die durch Kathepsine, Peptidasen, Carboxypeptidasen, α- und β-Glykosidasen, Lipasen, Phospholipasen, Phosphatasen, Phosphodiesterasen, Proteasen, Elastasen, Sulfatasen, Reduktasen und bakterielle Enzyme gespalten wird, steht,
- m: eine Zahl zwischen 1 und 80 ist,
wobei für den Fall, daß m eine Zahl zwischen 1 und 3 ist,
- A: ein Farbstoffmolekül mit mindestens einem Absorptionsmaximum zwischen 600 und 1200 nm, ein antibiotisch oder antizytostatisch wirksames Molekül, ein Biomolekül, ein nicht biologisches Makromolekül oder eine Verbindung B-(L-W)ₒ oder D-(L-W)ₒ darstellt, wobei
- D: ein nicht biologisches Makromolekül ist,
- B: ein Biomolekül ist,
- L: die oben genannte Bedeutung hat,
- W: ein antibiotisch oder antizytostatisch wirksames Molekül darstellt,
- o: eine Zahl zwischen 1 und 20 ist,
und wobei für den Fall, daß m eine Zahl zwischen 4 und 80 ist,
- A: ein Biomolekül, ein nicht biologisches Makromolekül oder eine Verbindung B-(L-W)ₒ oder D-(L-W)ₒ darstellt, wobei
- D, B, L, W und o: die oben genannten Bedeutungen haben.

Die besondere Eigenschaft hinsichtlich der In-vivo-Detektion der nahinfraroten Fluoreszenzemission der erfindungsgemäßen Verbindungen besteht darin, daß diese eine geringe bis gar keine Fluoreszenzemission aufweisen und erst nach Spaltung dieses Konstruktes bzw. Abspaltung des Farbstoffes vom Konstrukt am Zielort (z. B. Tumor, Entzündungen) eine Erhöhung des Fluoreszenzsignals auftritt. Die effektive Differenz des Fluoreszenzsignals zwischen zu detektierendem und umliegenden Gewebe wird demzufolge durch
a) die Konzentrationsdifferenz aufgrund pharmakokinetischer Mechanismen und
b) durch die Differenz in der Fluoreszenzquantenausbeute zum Zeitpunkt der Diagnostik
geprägt.

Es wurde gefunden, daß die Fluoreszenz der Farbstoffe gequencht wird, wenn ein Farbstoffmolekül an ein weiteres Molekül (Dimere) unter Erhalt der erfindungsgemäßen Verbindungen gekoppelt ist, d. h. es tritt eine äußerst geringe Fluoreszenzemission im Vergleich zum entsprechenden Farbstoffmolekül im ungebundenen Zustand auf. Es wurde darüberhinaus gefunden, daß ein vergleichbares Quenching auftritt, wenn andere Moleküle mit aromatischen Strukturen, welche sowohl Farbstoffe als auch Wirkstoffe (z. B. Zytostatika oder Antibiotika) sein können, mit dem Fluoreszenzfarbstoff gekoppelt sind. Überraschenderweise tritt ebenso ein Quenching bei Kopplung der Farbstoffe an Antikörper, Antikörperfragmente und Proteine auf.

Grundsätzlich müssen sich die Farbstoffe, die struktureller Bestandteil der erfindungsgemäßen Verbindungen sind, in ihrer monomeren unkonjugierten Form durch hohe molare Absorptionskoeffizienten und hohe Fluoreszenzquantenausbeuten auszeichnen.

Bevorzugte erfindungsgemäße Verbindungen der allgemeinen Formel I zeichnen sich dadurch aus, daß F und/oder A für einen
Polymethinfarbstoff, Tetrapyrrolfarbstoff, Tetraazapyrrolfarbstoff, Xanthinfarbstoff, Phenoxazinfarbstoff oder Phenothiazinfarbstoff stehen.

Besonders bevorzugt sind die Strukturen aus der Klasse der Polymethinfarbstoffe, da diese Absorptionsmaxima mit sehr hohen molaren Absorptionskoeffizienten im nahinfraroten Spektralbereich zwischen 700 und 1000 nm aufweisen (ε bis zu 300000 1 mol⁻¹ cm⁻¹), wie beispielsweise Cyaninfarbstoffe, Squariliumfarbstoffe und Croconiumfarbstoffe, sowie Merocyanin- und Oxonolfarbstoffe.

Ferner sind solche erfindungsgemäßen Verbindungen der allgemeinen Formel (I) bevorzugt, bei denen F und/oder A für einen

Cyaninfarbstoff der allgemeinen Formel II 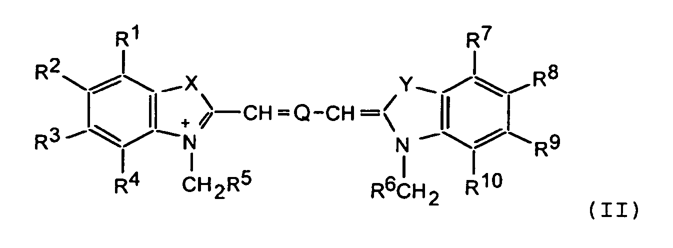 stehen,
worin
R¹ bis R⁴ und R⁷ bis R¹⁰ unabhängig voneinander für ein Fluor-, Chlor-, Brom-, Iodatom oder eine Nitrogruppe oder für einen Rest -COOE¹, -CONE¹E², -NHCOE¹, -NHCONHE¹, -NE¹E², -OE¹, -OSO₃E¹, -SO₃E¹, -SO₂NHE¹, -E¹,
wobei E¹ und E² unabhängig voneinander für ein Wasserstoffatom, eine gesättigte oder ungesättigte, verzweigte oder geradkettige C₁-C₅₀-Alkylkette, wobei die Kette oder Teile dieser Kette gegenbenenfalls eine oder mehrere aromatische oder gesättigte zyklische C₅-C₆- oder bizyklische C₁₀-Einheiten formen können, steht,und wobei die C₁-C₅₀-Alkylkette von 0 bis 15 Sauerstoffatomen und/oder von 0 bis 3 Carbonylgruppen unterbrochen ist und/oder mit 0 bis 5 Hydroxygruppen, 0 bis 5 Estergruppen, 0 bis 3 Carbongruppen, 0 bis 3 Aminogruppen, substituiert ist,
und wobei jeweils benachbarte Reste R₁ - R₄ und/oder R₇ - R₁₀ unter Bildung eines sechsgliedrigen aromatischen Kohlenstoffringes miteinander verknüpft sein können,
R⁵ und R⁶ unabhängig voneinander für einen Rest -E¹ mit der oben angegebenen Bedeutung oder für eine C₁-C₄-Sulfoalkylkette stehen,
und/oder R¹ bis R¹⁰ für eine Verknüpfung mit L stehen,
Q ein Fragment 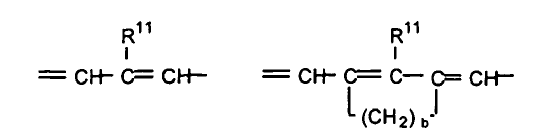 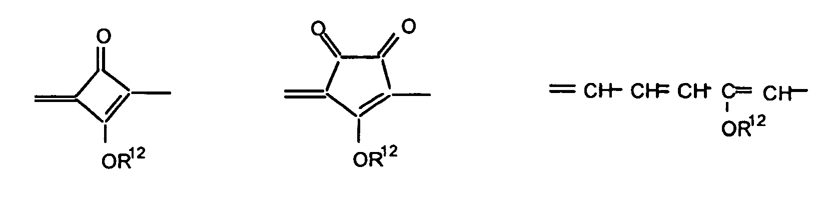 ist,
worin
R¹¹ für ein Wasserstoff-, Fluor-, Chlor-, Brom-, Iodatom oder eine Nitrogruppe oder einen Rest -NE¹E², -OE¹ oder -E¹, wobei E¹ und E² die oben angegebene Bedeutung haben oder für eine Verknüpfung mit L steht,
R¹² für ein Wasserstoffatom oder einen Rest E¹ mit der oben angegebenen Bedeutung steht,
b eine Zahl 0, 2 oder 3 bedeutet,
X und Y unabhängig voneinander O, S, -CH=CH- oder ein Fragment 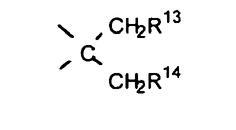 darstellt,
worin
R¹³ und R¹⁴ unabhängig voneinander für Wasserstoff, eine gesättigte oder ungesättigte, verzweigte oder geradkettige C₁ - C₁₀-Alkylkette, die durch bis zu 5 Sauerstoffatome unterbrochen und/oder mit bis zu 5 Hydroxygruppen substituiert sein kann, stehen, und wobei die Reste R¹³ und R¹⁴ unter Ausbildung eines 5- oder 6-gliedrigen Ringes miteinander verknüpft sein können.

Weiterer Gegenstand der Erfindung sind Verbindungen der allgemeinen Formel (I), in denen Farbstoffe mit einem therapeutisch wirksamen Molekül über eine physiologisch spaltbare Bindung verknüpft sind, oder Farbstoff und Wirkstoff über physiologisch spaltbare Bindungen an Biomoleküle oder nicht biologische Trägermoleküle gekoppelt sind.

Besonders bevorzugt sind Konstrukte, bei denen die Fluoreszenz des Farbstoffes im gekoppelten Zustand gequencht und die therapeutische Aktivität des Wirkmoleküls durch die Kopplung an Farbstoff bzw. Trägermolekül maskiert ist (Pro-Drug-Effekt). Die Spaltung der Bindung führt zu einer Erhöhung der Fluoreszenzemission bei gleichzeitiger Freisetzung der Aktivität des Wirkstoffes.

Wirkstoffe W und/oder A in der erfindungsgemäßen allgemeinen Formel (I) sind beispielsweise die im folgenden aufgeführten Verbindungen:

Antibiotika: Aclacinomycin, Actinomycin F₁, Anthramycin, Azaserin, Bleomycine, Cactinomycin, Carubicin, Carzinophilin, Chromomycine, Dactinomycin, Daunorubicin, Doxorubicin, Epirubicin, Mtiomycine, Mycophenolsäure, Nogalamycin, Olivomycine, Peplomycin, Plicamycin, Porfiromycin, Puromycin, Streptonigrin, Tubercidin, Zorubicin,
Folsäure-Analoga: Denopterin, Metothrexat, Pteropterin, Trimetrexat,
Pyrimidin-Analoga: Ancitabin, Azacitidin, 6-Azauridin, Carmofur, Cytarabin, Doxifluridin, Enocitabin, Floxuridin, 5-Fluor-Uracil,
Purin-Analoga: Fludarabin, 6-Mercaptopurin, Thiamiprin, Thioguanin und Derivate der genannten Verbindungen, alkylierende Substanzen: Alkylsulfonate, Aziridine, Ethylenimine, Methylmelamine, Nitroharnstoffe, Stickstofflostverbindungen,
hormonell wirksame Substanzen wie Androgene, Antiadrenale, Antiandrogene, Antiestrogene, Estrogene, LH-RH-Analoga und Progestogene,
sowie weitere zytostatisch wirksame Substanzen, wie Taxol und Taxol-Derivate.

Weitere Wirkstoffe sind photodynamisch aktive Substanzen, die sich durch das Vermögen auszeichnen, nach Anregung eine photosensibilisierende Wirkung durch Bildung zytotoxischen Singulettsauerstoffs und von Radikalen entfalten. Solche Verbindungen sind in erster Linie Tetrapyrrole bzw. Tetraazapyrrole, bespielsweise Porphyrine, Benzoporphyrine, Chlorine, Purpurine, Phthalocyanine, Naphthalocyanine und Derivate der genannten Verbindungen. Weitere Verbindungen sind expandierte Porphyrine, Porphycene und Oxazin- bzw. Phenoxazinfarbstoffe.

Die chemische Bindung, die gemäß der allgemeinen Formel (I) in der Linkerstruktur L enthalten ist, ist strukturell derart beschaffen, daß diese bei bestimmten physiologischen Parametern, durch die erkrankte Gewebe (Tumoren) charakterisiert sind und welche sich von normalen Gewebebereichen unterscheiden, gespalten wird.

Die Spaltung der erfindungsgemäßen Verbindungen kann durch Enzyme, die in den zu detektierenden Geweben (z. B.

Tumoren, bakteriellen Entzündungen) in erhöhter Konzentration vorliegen, erfolgen.

Gegenstand der Erfindung sind daher Verbindungen mit Linkerstrukturen L, die enzymatisch gespalten werden durch Kathepsine, Peptidasen, Carboxypeptidasen, α- und β-Glukosidasen, Lipasen, Oxidasen, Phospholipasen, Phosphatasen, Phosphodiesterasen, Proteasen, Elastasen, Sulfatasen, Reduktasen, Transferasen und bakterielle Enzyme, beispielsweise Penicillin-Amidasen sowie β-Lactamasen, P. D. Senter et al., Bioconjugate Chem. 6 (1995), 389-94).

Bevorzugte enzymatisch spaltbare Strukturen sind kurzkettige Peptidsequenzen, wie beispielsweise Sequenzen, die die Aminosäuresequenz Val-Leu-Lys enthalten.

Die Kinetik, die zu einer Anreicherung im zu detektierenden Gewebe bzw. zu einem entsprechenden Konzentrationsgradienten zu einem bestimmten Zeitpunkt nach Applikation führt, muß sowohl mit der Kinetik der Spaltung der erfindungsgemäßen Verbindungen als auch der Kinetik des Abtransportes des freigesetzten Farbstoffmoleküls korrelieren und zu einem synergistischen Effekt führen.

Weitere bevorzugte erfindungsgemäße Verbindungen der allgemeinen Formel (I) zeichnen sich dadurch aus, daß A und/oder B für einen Antikörper, deren Konjugate und Fragmente, spezifische Peptide und Proteine, Rezeptoren, Enzyme, Enzymsubstrate, Nukleotide, natürliche oder synthetische Ribonukleinsäuren oder Desoxyribonukleinsäuren oder deren chemische Modifikationen, wie Aptamere oder Antisenseoligonukleotide, Lipoproteine, Lectine, Kohlenhydrate, Mono-, Di- oder Trisaccharide, lineare oder verzweigte Oligo- oder Polysaccharide oder -saccharidderivate oder für ein Dextran steht.

Ferner sind die erfindungsgemäßen Verbindungen der allgemeinen Formel (I) bevorzugt, in denen D Polyethylenglykol, Polypropylenglykol, Polylysin oder Polylysin-Dendrimere oder deren Derivate darstellen.

Die Verknüpfung der Strukturelemente A, D, B, L und W erfolgt entweder direkt oder über übliche funktionelle Gruppen. Solche Gruppen sind beispielsweise Ester, Ether, sekundäre und tertiäre Amine, Amide, Thioharnstoff-, Harnstoff-, Carbamatgruppen oder Maleimidostrukturen.

Ein weiterer Gegenstand der Erfindung ist die Verwendung der erfindungsgemäßen Verbindungen der allgemeinen Formel I zur In-vivo-Diagnostik erkrankter Gewebebereiche mittels NIR-Strahlung sowie zur Therapie erkrankter Gewebebereiche.

Gegenstand der Erfindung ist ferner ein optisches Diagnostikum zur In-vivo-Diagnostik erkrankter Gewebebereiche mittels NIR-Strahlung, welches mindestens eine erfindungsgemäße Verbindung der allgemeinen Formel (I) enthält.

Diese Mittel werden nach den dem Fachmann bekannten Methoden hergestellt, ggf. unter Verwendung üblicher Hilfs- und/oder Trägerstoffe sowie Verdünnungsmittel und dergleichen. Dazu gehören physiologisch verträgliche Elektrolyte, Puffer, Detergenzien und Substanzen zur Anpassung der Osmolarität sowie zur Verbesserung der Stabilität und Löslichkeit. Durch die in der Pharmazie gebräuchlichen Maßnahmen ist für die Sterilität der Zubereitungen bei der Herstellung und insbesondere vor der Applikation zu sorgen.

Die Synthese der Farbstoffe F und A erfolgt nach literaturbekannten Methoden, z. B.
F.M. Hamer in The Cyanine Dyes and Related Compounds, John Wiley and Sons, New York, 1964;
J. Fabian et al., Chem. Rev. 92 (1992) 1197;
L.A. Ernst et al., Cytometrie 10 (1989) 3-10;
P.L. Southwick et al., Cytometrie 11 (1990) 418-430;
R. B. Mujumdar et al., Bioconjugate Chem. 4 (1993)105-11;
E. Terpetschnig et al., Anal. Biochem. 217 (1994)197-204;
J. S. Lindsey et al., Tetrahedron 45 (1989)4845-66, EP-0591820 Al;
L. Strekowski et al., J. Heterocycl. Chem. 33 (1996) 1685-1688;
S. R. Mujumdar et al., Bioconjugate Chem. 7 (1996) 356-362;
M. Lipowska et al., Synth. Commun. 23 (1993) 3087-94;
E. Terpetschnig et al., Anal. Chim. Acta 282 (1993) 633-641;
M. Matsuoka und T. Kitao, Dyes Pigm. 10 (1988) 13-22 und N. Narayanan und G. Patronay, I. Org. Chem. 60 (1995) 2361-95.

Die Farbstoffe werden in Anlehnung an literaturbekannte Methoden mit Substituenten synthetisiert, die durch Kathepsine, Peptidasen, Carboxypeptidasen, α- und β-Glykosidasen, Lipasen, Phospholipasen, Phosphatasen, Phosphodiesterasen, Proteasen, Elastasen, Sulfatasen, Reduktasen und bakterielle Enzyme spaltbare Bindungen enthalten, oder aus denen solche Bindungen nach Kopplung entstehen; z. B. nach
B. M. Mueller et al., Bioconjugate Chem. 1 (1990) 325-330;
K. Srinivasachar und D. M. Neville, Biochemistry 28(1989) 2501-09;
D. M. Neville et al., J. Biol. Chem. 264 (1989) 14653-61; T. Kaneko et al., Bioconjugate Chem. 2 (1991), 133-41;
B. A. Froesch et al., Cancer Immunol. Immunother. 42 (1996), 55-63 und
J. V. Crivello et al., J. Polymer Sci: Part A: Polymer Chem. 34 (1996) 3091-3102.

Die nachfolgenden Beispiele erläutern die Erfindung: Beispiele:

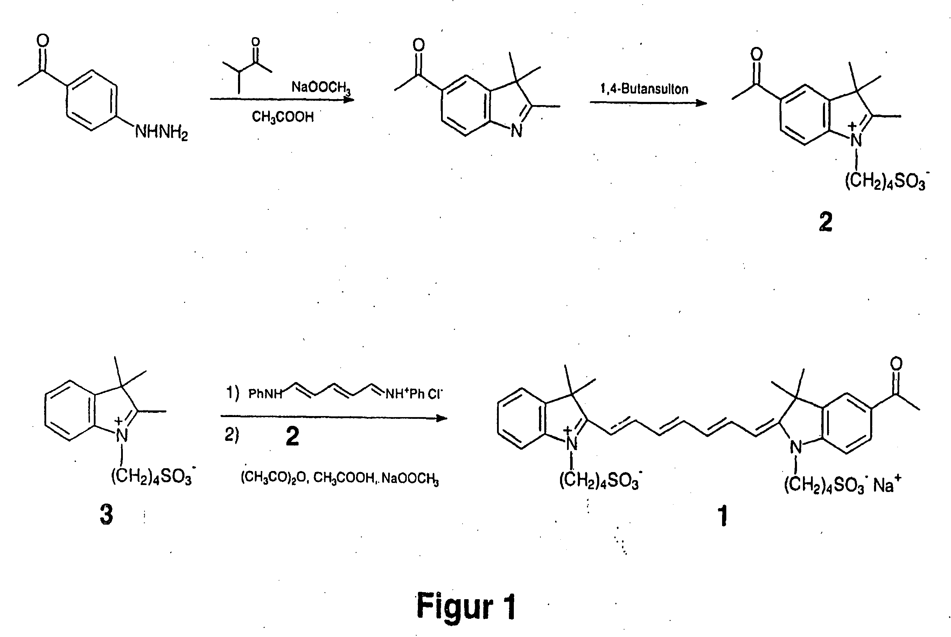

### 1. Synthese von 5-(1-Oxoethyl)-1,1'-(4-sulfobutyl)-indotricarbocyanin-natriumsalz 1 (Figur 1)

4-Hydrazinophenylmethylketon wird aus 4-Aminophenylmethylketon durch Diazotierung und Reduktion mit SnCl₂ synthetisiert (in Anlehnung an T. Górecki et al., J. Heterocyclic Chem. 33 (1996) 1871-76).
4,8 g (32 mmol) 4-Hydrazinophenylmethylketon, 5,4 g Natriumacetat und 3,9 g (45 mmol) 3-Methyl-2-butanon werden in 40 ml Essigsäure 1 h bei Raumtemperatur und 4 h bei 120°C gerührt. Das Reaktionsgemisch wird im Vakuum eingedampft, in 300 ml Dichlormethan aufgenommen und die organische Phase mit ges. NaCl-Lsg. gewaschen. Nach Trocknen über MgSO₄ erhält man 7,5 g eines braunen Öls. Dieses wird mit 6,5 g (48 mmol) 1,4-Butansulton 5 h auf 140°C erhitzt, nach dem Abkühlen mit Aceton verrührt und der ausgefallene Feststoff chromatographisch (RP C-18, Laufmittel Methanol/Wasser) gereinigt. Ausbeute: 2,5 g (23%) 5-(1-Oxoethyl)-1-(4-Sulfobutyl)-2,3,3-trimethyl-3H-indolenin **2**.
Zur Darstellung des Farbstoffes 1 werden 0,5 g (1,7 mmol) 1-(9-Sulfobutyl)-2,3,3-trimethyl-3H-indolenin **3** mit 0,47 g (1,6 mmol) Glutaconaldehyddianilhydrochlorid in 10 ml Essigsäureanhydrid 30 min bei 120°C gerührt. Nach Abkühlen wird mit 0,6 g (1,8 mmol) **2**, 10 ml Essigsäureanhydrid, 4 ml Essigsäure und 0,5 g Natriumacetat versetzt und 30 min auf 120°c erhitzt. Die tiefblaue Lösung wird abgekühlt, mit 200 ml Ether verrührt und der ausgefallene Feststoff abfiltriert.
Nach chromatographischer Reinigung (RP C-18, Laufmittel Methanol/Wasser) und Gefriertrocknung erhält man 0,3 g (26%) Produkt **1**.

### Elementaranalyse:

Ber.: C 61,99 H 6,33 N 3,91 S 8,95
Gef.: C 61,73 H 6,49 N 3,80 S 8,78
Absorption: λₘₐₓ (H₂O) = 748 nm (ε= 148000 1 mol⁻¹ cm⁻¹)

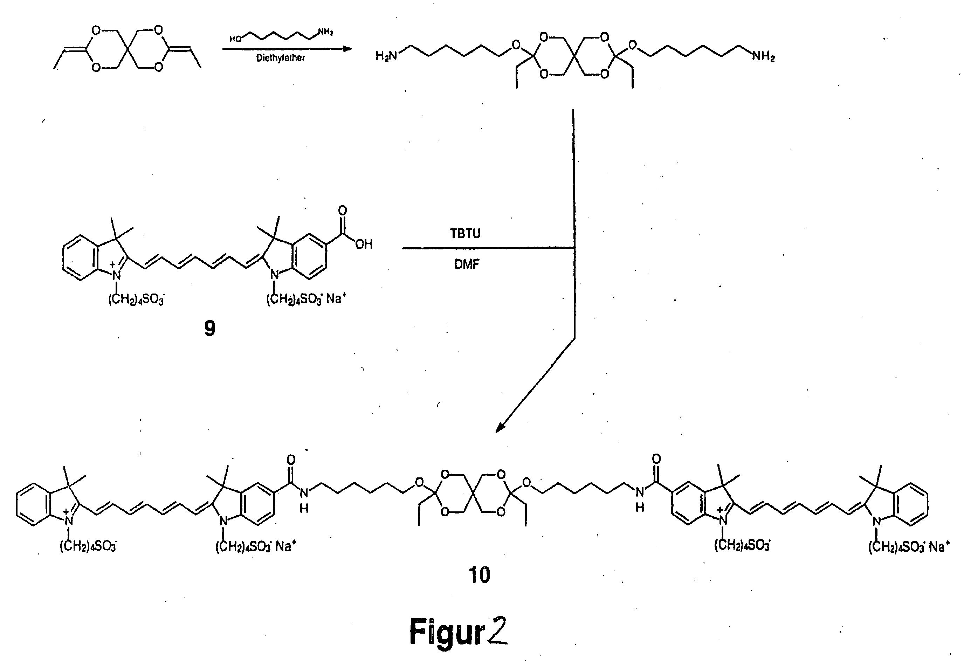

### 2. Darstellung des symmetrischen Spirodimers 10 (Figur 2)

0,1 g (0,47 mmol) 3,9-Diethyliden-2,4,8,10-tetraoxaspiro-[5.5]undecan (nach M. Crivello et al., J. Polymer Sci.: Part A: Polymer Chem. 34 (1996) 3091-3102 synthetisiert) und 0,11 g (0,94 mmol) 6-Amino-1-hexanol werden in 15 ml Diethylether 24 h bei Raumtemperatur gerührt und das Lösungsmittel im Vakuum verdampft. Der Rückstand wird an der Ölpumpe getrocknet und ohne weitere Reinigung umgesetzt.
0,2 g (0,28 mmol) 5-Carboxy-bis-1,1'-(9-sulfobutyl)-indotricarbocyanin-natriumsalz **9** werden in 15 ml Dichlormethan zusammen mit 0,09 g (0,28 mmol) TBTU und 30 mg Triethylamin 30 min gerührt und mit 0,06 g (0,14 mmol) o. g. Spiroverbindung in 2 ml Dichlormethan versetzt. Nach 18 h Rühren bei Raumtemperatur wird das Produkt mit Diethylether ausgefällt und chromatographisch gereinigt (RP C-18, Laufmittel Methanol/10 mM Phosphatpuffer pH 8). Nach Gefriertrocknung werden die Salze mit Methanol/Dichlormethan ausgefällt. Man erhält 68 mg (26%) Produkt **10**.

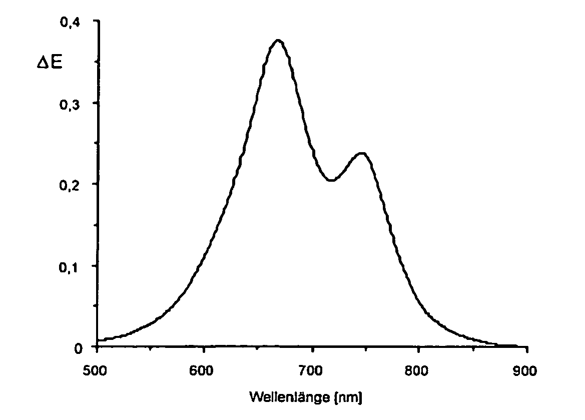

### VIS/NIR-Absorptionspektrum von 10 (5 µmol/l in Phophatpuffer pH 8)

Fluoreszenzquantenausbeute Q = 0,2 % (5 µmol/l in Phophatpuffer pH 8; bezogen auf Indocyaningrün als Standard).

## Patentansprüche

1. Verbindungen der allgemeinen Formel I
(F-L)ₘ-A (I)
worin
F für ein Cyanin-, Squarilium-, Croconium-, Merocyanin- oder Oxonolfarbstoffmolekül mit mindestens einem Absorptionsmaximum zwischen 600 und 1200 nm steht,
L für eine Linkerstruktur, welche eine enzymatisch spaltbare Bindung enthält, steht, die durch Kathepsine, Peptidasen, Carboxypeptidasen, α- und β-Glykosidasen, Lipasen, Phospholipasen, Phosphatasen, Phosphodiesterasen, Proteasen, Elastasen, Sulfatasen, Reduktasen und bakterielle Enzyme gespalten wird,
m eine Zahl zwischen 1 und 80 ist,
wobei für den Fall, daß m eine Zahl zwischen 1 und 3 ist,
A ein Farbstoffmolekül mit mindestens einem Absorptionsmaximum zwischen 600 und 1200 nm, ein antibiotisch oder antizytostatisch wirksames Molekül, ein Biomolekül, ein nicht biologisches Makromolekül oder eine Verbindung B-(L-W)ₒ oder D-(L-W)ₒ darstellt, wobei
D ein nicht biologisches Makromolekül ist,
B ein Biomolekül ist,
L die oben genannte Bedeutung hat,
W ein antibiotisch oder antizytostatisch wirksames Molekül darstellt,
o eine Zahl zwischen 1 und 20 ist,
und wobei für den Fall, daß m eine Zahl zwischen 4 und 80 ist,
A ein Biomolekül, ein nicht biologisches Makromolekül oder eine Verbindung B-(L-W)ₒ oder D-(L-W)ₒ darstellt, wobei
D, B, L, W und o die oben genannten Bedeutungen haben.

2. Verbindungen der allgemeinen Formel I
(F-L)ₘ-A (I)
worin
F für ein Cyanin-, Squarilium-, Croconium-, Merocyanin- oder Oxonolfarbstoffmolekül mit mindestens einem Absorptionsmaximum zwischen 600 und 1200 nm steht,
L für eine enzymatisch spaltbare Linkerstruktur steht, die aus einer kurzkettigen Peptidsequenz besteht,
m eine Zahl zwischen 1 und 80 ist,
wobei für den Fall, daß m eine Zahl zwischen 1 und 3 ist,
A ein Farbstoffmolekül mit mindestens einem Absorptionsmaximum zwischen 600 und 1200 nm, ein antibiotisch oder antizytostatisch wirksames Molekül, ein Biomolekül, ein nicht biologisches Makromolekül oder eine Verbindung B-(L-W)ₒ oder D-(L-W)ₒ darstellt, wobei
D ein nicht biologisches Makromolekül ist,
B ein Biomolekül ist,
L die oben genannte Bedeutung hat,
W ein antibiotisch oder antizytostatisch wirksames Molekül darstellt,
o eine Zahl zwischen 1 und 20 ist,
und wobei für den Fall, daß m eine Zahl zwischen 4 und 80 ist,
A ein Biomolekül, ein nicht biologisches Makromolekül oder eine Verbindung B-(L-W)ₒ oder D-(L-W)ₒ darstellt, wobei
D, B, L, W und o die oben genannten Bedeutungen haben.

3. Verbindungen nach Anspruch 1 oder Anspruch 2, **dadurch gekennzeichnet, daß** in den allgemeinen Formeln (I) A für einen Polymethinfarbstoff, Tetrapyrrolfarbstoff, Tetraazapyrrolfarbstoff, Xanthinfarbstoff, Phenoxazinfarbstoff oder Phenothiazinfarbstoff steht.

4. Verbindungen nach mindestens einem der vorhergehenden Ansprüche, **dadurch gekennzeichnet, daß** in der allgemeinen Formel (I) A für einen Cyanin-, Squarilium-, Croconium-, Merocyanin- oder Oxonolfarbstoff stehen.

5. Verbindungen nach mindestens einem der vorhergehenden Ansprüche, **dadurch gekennzeichnet, daß** in der allgemeinen Formel (I) F und/oder A für einen Cyaninfarbstoff der allgemeinen Formel (II) 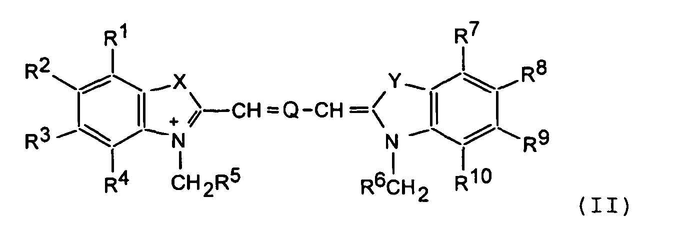 stehen,
worin
R¹ bis R⁴ und R⁷ bis R¹⁰ unabhängig voneinander für ein Fluor-, Chlor-, Brom-, Iodatom oder eine Nitrogruppe oder für einen Rest -COOE¹, -CONE¹E², -NHCOE¹, -NHCONHE¹, -NE¹E², -OE¹, -OSO₃E¹, -SO₃E¹, -SO₂NHE¹, -E¹,
wobei E¹ und E² unabhängig voneinander für ein Wasserstoffatom, eine gesättigte oder ungesättigte, verzweigte oder geradkettige C₁-C₅₀-Alkylkette, wobei die Kette oder Teile dieser Kette gegenbenenfalls eine oder mehrere aromatische oder gesättigte zyklische C₅-C₆- oder bizyklische C₁₀-Einheiten formen können, steht,und wobei die C₁-C₅₀-Alkylkette von 0 bis 15 Sauerstoffatomen und/oder von 0 bis 3 Carbonylgruppen unterbrochen ist und/oder mit 0 bis 5 Hydroxygruppen, 0 bis 5 Estergruppen, 0 bis 3 Carboxygruppen bzw. 0 bis 3 Aminogruppen substituiert ist,
und wobei jeweils benachbarte Reste R₁ - R₄ und/oder R₇ - R₁₀ unter Bildung eines sechsgliedrigen aromatischen Kohlenstoffringes miteinander verknüpft sein können,
R⁵ und R⁶ unabhängig voneinander für einen Rest -E¹ mit der oben angegebenen Bedeutung oder für eine C₁-C₄-Sulfoalkylkette stehen,
und/oder R¹ bis R¹⁰ für eine Verknüpfung mit L stehen,
Q ein Fragment 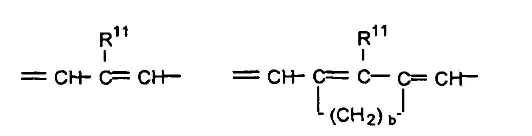 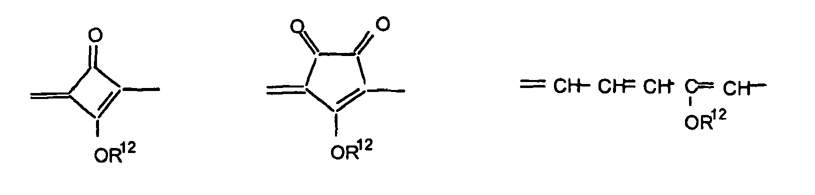
ist,
worin
R¹¹ für ein Wasserstoff-, Fluor-, Chlor-, Brom-, Iodatom oder eine Nitrogruppe oder einen Rest -NE¹E², -OE¹ oder -E¹, wobei E¹ und E² die oben angegebene Bedeutung haben oder für eine Verknüpfung mit L steht,
R¹² für ein Wasserstoffatom oder einen Rest E¹ mit der oben angegebenen Bedeutung steht,
b eine Zahl 0, 2 oder 3 bedeutet,
X und Y unabhängig voneinander Reste O, S, -CH=CH-oder ein Fragment 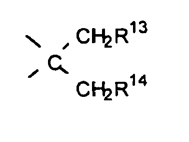
darstellen,
worin
R¹³ und R¹⁴ unabhängig voneinander für Wasserstoff, eine gesättigte oder ungesättigte, verzweigte oder geradkettige C₁ - C₁₀-Alkylkette, die durch bis zu 5 Sauerstoffatome unterbrochen und/oder mit bis zu 5 Hydroxygruppen substituiert sein kann, stehen, und wobei die Reste R¹³ und R¹⁴ unter Ausbildung eines 5- oder 6-gliedrigen Ringes miteinander verknüpft sein können.

6. Verbindungen nach mindestens einem der vorhergehenden Ansprüche, **dadurch gekennzeichnet, daß** in der allgemeinen Formel (I) W oder A für Antibiotika, Folsäure-Analoga, Pyrimidin-Analoga, Purin-Analoga, hormonell wirksame Substanzen sowie weitere cytostatisch wirksame Substanzen stehen.

7. Verbindungen nach Anspruch 2, **dadurch gekennzeichnet, daß** in der allgemeinen Formel (I) L für eine enzymatisch spaltbare Linkerstruktur, bestehend aus einer kurzkettigen Peptidsequenz, steht, die durch Kathepsine, Peptidasen, Carboxypeptidasen, α- und β-Glykosidasen, Lipasen, Phospholipasen, Phosphatasen, Phosphodiesterasen, Proteasen, Elastasen, Sulfatasen, Reduktasen und bakterielle Enzyme gespalten wird.

8. Verbindungen nach mindestens einem der vorhergehenden Ansprüche, **dadurch gekennzeichnet, daß** in der allgemeinen Formel (I)
A und/oder B für einen Antikörper, deren Konjugate und Fragmente, spezifische Peptide und Proteine, Rezeptoren, Enzyme, Enzymsubstrate, Nukleotide, natürliche oder synthetische Ribonukleinsäuren oder Desoxyribonukleinsäuren oder deren chemische Modifikationen, wie Aptamere oder Antisenseoligonukleotide, Lipoproteine, Lectine, Kohlenhydrate, Mono-, Di- oder Trisaccharide, lineare oder verzweigte Oligo- oder Polysaccharide oder -saccharidderivate oder für ein Dextran steht.

9. Verbindungen nach mindestens einem der vorhergehenden Ansprüche, **dadurch gekennzeichnet, daß** in der allgemeinen Formel (I) D für Polyethylenglykol, Polypropylenglykol, Polylysin oder Polylysin-Dendrimere oder deren Derivate steht.

10. Optisches Diagnostikum zur In-vivo-Diagnostik erkrankter Gewebebereiche mittels NIR-Strahlung, **dadurch gekennzeichnet, daß** es mindestens eine Verbindung nach Anspruch 1 zusammen mit den üblichen Hilfs- und /oder Trägerstoffen sowie Verdünnungsmitteln enthält.

11. Verbindungen nach Anspruch 1, **dadurch gekennzeichnet, daß** in der allgemeinen Formel (I) L für eine enzymatisch spaltbare Linkerstruktur steht, bestehend aus einer kurzkettigen Peptidsequenz.

## Claims

1. Compounds of general formula I
(F-L)ₘ-A (I),
in which
F is a cyanine, squarilium, croconium, merocyanine or oxonol dye molecule with at least one absorption maximum of between 600 and 1200 nm,
L is a linker structure which contains an enzymatically cleavable bond, which is cleaved by cathepsins, peptidases, carboxypeptidases, α- and β-glycosidases, lipases, phospholipases, phosphatases, phosphodiesterases, proteases, elastases, sulfatases, reductases and bacterial enzymes,
m is a number between 1 and 80,
wherein if m is a number between 1 and 3,
A represents a dye molecule with at least one absorption maximum of between 600 and 1200 nm, an antibiotically or anticytostatically active molecule, a biomolecule, a non-biological macromolecule or a compound B-(L-W)ₒ or D-(L-W)ₒ, wherein
D is a non-biological macromolecule,
B is a biomolecule,
L has the above-mentioned meaning,
W represents an antibiotically or anticytostatically active molecule,
o is a number between 1 and 20,
and wherein if m is a number between 4 and 80,
A represents a biomolecule, a non-biological macromolecule or a compound B-(L-W)ₒ or D-(L-W)ₒ,
wherein
D, B, L, W and o have the above-mentioned meanings.

2. Compounds of general formula I
(F-L)ₘ-A (I),
in which
F is a cyanine, squarilium, croconium, merocyanine or oxonol dye molecule with at least one absorption maximum of between 600 and 1200 nm,
L is an enzymatically cleavable linker structure which consists of a short-chain peptide sequence,
m is a number between 1 and 80,
wherein if m is a number between 1 and 3,
A represents a dye molecule with at least one absorption maximum of between 600 and 1200 nm, an antibiotically or anticytostatically active molecule, a biomolecule, a non-biological macromolecule or a
compound B-(L-W)ₒ or D-(L-W)ₒ, wherein
D is a non-biological macromolecule,
B is a biomolecule,
L has the above-mentioned meaning,
W represents an antibiotically or anticytostatically active molecule,
o is a number between 1 and 20,
and wherein if m is a number between 4 and 80,
A represents a biomolecule, a non-biological macromolecule or a compound B-(L-W)ₒ or D-(L-W)ₒ,
wherein
D, B, L, W and o have the above-mentioned meanings.

3. Compounds according to claim 1 or claim 2, **characterized in that** in general formula (I) A is a polymethine dye, tetrapyrrole dye, tetraazapyrrole dye, xanthine dye, phenoxazine dye or phenothiazine dye.

4. Compounds according to at least one of the preceding claims, **characterized in that** in general formula (I) A is a cyanine, squarilium, croconium, merocyanine or oxonol dye.

5. Compounds according to at least one of the preceding claims, **characterized in that** in general formula (I) F and/or A are a cyanine dye of general formula (II) 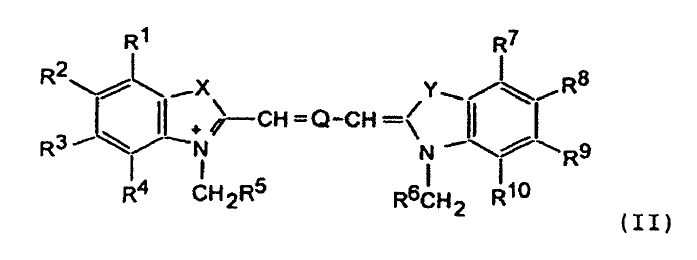 in which
R¹ to R⁴ and R⁷ to R¹⁰, independently of one another, are a fluorine, chlorine, bromine, iodine atom or a nitro group or a radical -COOE¹, -CONE¹E², -NHCOE¹, -NHCONHE¹, -NE¹E², -OE¹, -OSO₃E¹-, -SO₃E¹, -SO₂NHE¹, -E¹, wherein E¹ and E², independently of one another, are a hydrogen atom, a saturated or unsaturated, branched or straight-chain C₁-C₅₀ alkyl chain, wherein the chain or parts of this chain optionally can form one or more aromatic or saturated cyclic C₅-C₆ units or bicyclic C₁₀ units, and wherein the C₁-C₅₀ alkyl chain is interrupted by 0 to 15 oxygen atoms and/or 0 to 3 carbonyl groups and/or is substituted with 0 to 5 hydroxy groups, 0 to 5 ester groups, 0 to 3 carboxy groups or 0 to 3 amino groups, and wherein in each case adjacent radicals R¹-R⁴ and/or R⁷-R¹⁰ can be linked with one another with the formation of a six-membered aromatic carbon ring,
R⁵ and R⁶, independently of one another, are a radical -E¹ with the above-mentioned meaning or a C₁-C₄ sulfoalkyl chain, and/or R¹ to R¹⁰ are a linkage with L,
Q is a fragment 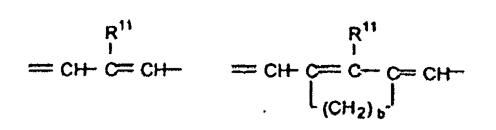 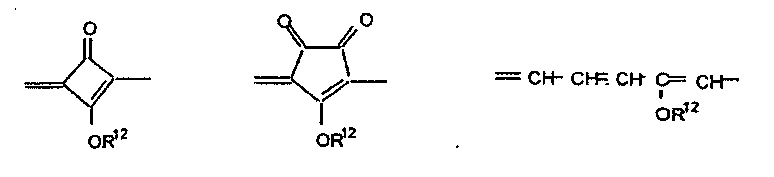 in which
R¹¹ is a hydrogen, fluorine, chlorine, bromine or iodine atom or a nitro group or a radical -NE¹E², -OE¹ or -E¹,
wherein E¹ and E² have the above-mentioned meaning or R¹¹ is a linkage with L,
R¹² is a hydrogen atom or a radical E¹ with the above-mentioned meaning,
b is a number 0, 2 or 3,
X and Y, independently of one another, represent radicals O, S, -CH=CH- or a fragment 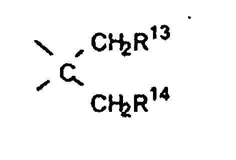 in which
R¹³ and R¹⁴, independently of one another, are hydrogen, a saturated or unsaturated, branched or straight-chain C₁-C₁₀ alkyl chain, which can be interrupted by up to 5 oxygen atoms and/or substituted with up to 5 hydroxy groups, and wherein radicals R¹³ and R¹⁴ can be linked with one another with the formation of a 5- or 6-membered ring.

6. Compounds according to at least one of the preceding claims, **characterized in that** in general formula (I), W or A is an antibiotic, a folic acid analog, a pyrimidine analog, a purine analog, a hormonally active substance or another cytostatically active substance.

7. Compounds according to claim 2, **characterized in that** in general formula (I), L is an enzymatically cleavable linker structure consisting of a short-chain peptide sequence and cleaved by cathepsins, peptidases, carboxypeptidases, α- and β-glycosidases, lipases, phospholipases, phosphatases, phosphodiesterases, proteases, elastases, sulfatases, reductases and bacterial enzymes.

8. Compounds according to at least one of the preceding claims, **characterized in that** A and/or B is an antibody, its conjugates and fragments, specific peptides and proteins, receptors, enzymes, enzyme substrates, nucleotides, natural or synthetic ribonucleic acids or deoxyribonucleic acids or their chemical modifications, such as aptamers or antisense oligonucleotides, lipoproteins, lectins, carbohydrates, mono-, di- or trisaccharides, linear or branched oligosaccharides or polysaccharides or -saccharide derivatives or a dextran.

9. Compounds according to at least one of the preceding claims, **characterized in that** in general formula (I), D is polyethylene glycol, polypropylene glycol, polylysine or polylysine dendrimers or derivatives thereof.

10. An optical diagnostic agent for in vivo diagnosis of diseased tissue areas with use of NIR radiation, containing at least one compound according to claim 1 in combination with commonly used adjuvants and/or vehicles and also diluents.

11. Compounds according to claim 1, **characterized in that** in general formula (I), L is an enzymatically cleavable linker structure consisting of a short-chain peptide sequence.

## Revendications

1. Composés de formule générale I
(F-L)ₘ-A (I)
dans laquelle
F représente une molécule de colorant cyanine, squarylium, croconium, mérocyanine ou oxonal présentant au moins un maximum d'absorption entre 600 et 1200 nm,
L représente une structure de lien qui contient une liaison dissociable par voie enzymatique, qui est dissociée par la cathepsine, les peptidases, les carboxypeptidases, les α-glycosidases et les β-glycosidases, les lipases, les phospholipases, les phosphatases, les phosphodiestérases, les protéases, les élastases, les sulfatases, les réductases et les enzymes bactériennes,
m est un nombre entre 1 et 80,
où, dans le cas où m serait un nombre entre 1 et 3,
A est une molécule de colorant présentant au moins un maximum d'absorption entre 600 et 1200 nm, une molécule à activité antibiotique ou anticytostatique, une biomolécule, une macromolécule non biologique ou une liaison B-(L-W)ₒ ou D-(L-W)ₒ, où
D est une macromolécule non biologique,
B est une biomolécule,
L a la signification susmentionnée,
W est une molécule à activité antibiotique
ou anticytostatique,
o est un nombre entre 1 et 20,
et où, dans le cas où m serait un nombre entre 4 et 80,
A est une biomolécule, une macromolécule non biologique ou un composé B-(L-W)ₒ ou D-(L-W)ₒ, où
D, B, L, W et o ont les significations susmentionnées.

2. Composés de formule générale I
(F-L)ₘ-A (I)
dans laquelle
F représente une molécule de colorant cyanine, squarylium, croconium, mérocyanine ou oxonal présentant au moins un maximum d'absorption entre 600 et 1200 nm,
L représente une structure de lien dissociable par voie enzymatique qui consiste en une séquence peptidique à courte chaîne,
m est un nombre entre 1 et 80,
où, dans le cas où m serait un nombre entre 1 et 3,
A est une molécule de colorant présentant au moins un maximum d'absorption entre 600 et 1200 nm, une molécule à activité antibiotique ou anticytostatique, une biomolécule, une macromolécule non biologique ou une liaison B-(L-W)ₒ ou D- (L-W)ₒ, où
D est une macromolécule non biologique,
B est une biomolécule,
L a la signification susmentionnée,
W est une molécule à activité antibiotique ou anticytostatique,
o est un nombre entre 1 et 20,
et où, dans le cas où m serait un nombre entre 4 et 80,
A est une biomolécule, une macromolécule non biologique ou un composé B-(L-W)ₒ ou D-(L-W)ₒ, où
D, B, L, W et o ont les significations susmentionnées.

3. Composés selon la revendication 1 ou la revendication 2, **caractérisés en ce que** dans les formules générales (I), A représente un colorant polyméthine, un colorant tétrapyrrole, un colorant tétraazapyrrole, un colorant xanthine, un colorant phénoxazine ou un colorant phénothiazine.

4. Composés selon au moins l'une quelconque des revendications précédentes, **caractérisés en ce que** dans la formule générale (I), A représente un colorant cyanine, squarylium, croconium, mérocyanine ou oxonol.

5. Composés selon au moins l'une quelconque des revendications précédentes, **caractérisés en ce que** dans la formule générale (I), F et/ou A représentent un colorant cyanine de formule générale (II), 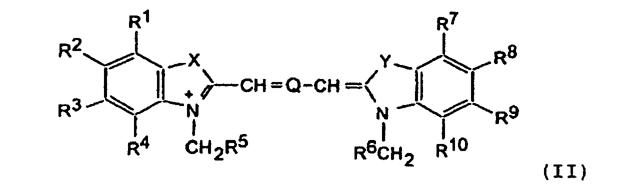 dans laquelle
R¹ à R⁴ et R⁷ à R¹⁰ représentent, indépendamment l'un de l'autre, un atome de fluor, de chlore, de brome, d'iode ou un groupe nitro ou un radical -COOE¹, CONE¹E², -NHCOE¹, -NHCONHE¹, NE¹E², -OE¹, -OSO₃E¹, -SO₃E¹, -SO₂NHE¹, -E¹,
où E¹ et E² représentent indépendamment l'un de l'autre un atome d'hydrogène, une chaîne alkyle en C₁ à C₅₀ saturée ou insaturée, ramifiée ou linéaire, la chaîne ou une partie de cette chaîne pouvant le cas échéant former une ou plusieurs unités aromatiques ou saturées cycliques en C₅ à C₆ ou bicycliques en C₁₀, et où la chaîne alkyle en C₁ à C₅₀ est interrompue par 0 à 15 atomes d'oxygène et/ou par 0 à 3 groupes carbonyle et/ou substituée par 0 à 5 groupes hydroxy, 0 à 5 groupes ester, 0 à 3 groupes carboxy ou, selon le cas, 0 à 3 groupes amino,
et où des radicaux R¹ à R⁴ et/ou R⁷ à R¹⁰ à chaque fois adjacents peuvent être liés l'un à l'autre en formant un cycle carboné aromatique à six chaînons,
R⁵ et R⁶ représentent indépendamment l'un de l'autre un radical -E¹ ayant la signification susmentionnée ou une chaîne sulfoalkyle en C₁ à C₄,
et/ou R¹ à R¹⁰ représentent un lien avec L,
Q représente un fragment 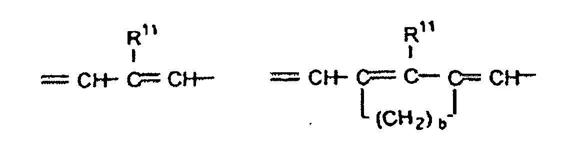 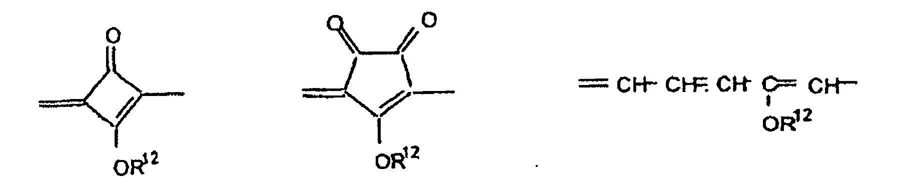 où
R¹¹ représente un atome d'hydrogène, de fluor, de chlore, de brome, d'iode ou un groupe nitro ou un radical -NE¹E², -OE¹ ou -E¹, où E¹ et E² ont la signification susmentionnée ou représente une liaison avec L,
R¹² représente un atome d'hydrogène ou un radical E¹ avec la signification susmentionnée,
b représente un nombre 0, 2 ou 3,
X et Y représentent, indépendamment l'un de l'autre, les radicaux O, S, -CH=CH- ou un fragment 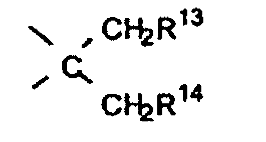 dans lequel R¹³ et R¹⁴ représentent, indépendamment l'un de l'autre, hydrogène, une chaîne alkyle en C₁ à C₁₀ saturée ou insaturée, ramifiée ou linéaire, qui peut être interrompue par jusqu'à 5 atomes d'oxygène et/ou substituée par jusqu'à 5 groupes hydroxy, et où les radicaux R¹³ et R¹⁴ peuvent être liés l'un avec l'autre en formant un cycle à 5 ou 6 chaînons.

6. Composés selon au moins l'une quelconque des revendications précédentes, **caractérisés en ce que** dans la formule générale (I), W ou A représente des antibiotiques, des analogues d'acide folique, des analogues de pyrimidine, des analogues de purine, des substances à activité hormonale ainsi que d'autres substances à activité cytostatique.

7. Composés selon la revendication 2, **caractérisés en ce que** dans la formule générale (I), L représente une structure de lien dissociable par voie enzymatique, consistant en une séquence peptidique à courte chaîne, qui est dissociée par la cathepsine, les peptidases, les carboxypeptidases, les α-glycosidases et les β-glycosidases, les lipases, les phospholipases, les phosphatases, les phosphodiesterases, les protéases, les élastases, les sulfatases, les réductases et les enzymes bactériennes.

8. Composés selon au moins l'une quelconque des revendications précédentes, **caractérisés en ce que** dans la formule générale (I)
A et/ou B représentent un anticorps, ses conjugués et fragments, des peptides et protéines spécifiques, des récepteurs, des enzymes, des substrats d'enzyme, des nucléotides, des acides ribonucléiques ou désoxyribonucléiques naturels ou synthétiques ou leurs modifications chimiques, telles que les aptamères ou les oligonucléotides antisens, des lipoprotéines, des lectines, des hydrates de carbone, des monosaccharides, des disaccharides ou des trisaccharides, des oligosaccharides ou des polysaccharides ou des dérivés de polysaccharides linéaires ou ramifiés ou un dextran.

9. Composés selon au moins l'une quelconque des revendications précédentes, **caractérisés en ce que** dans la formule générale (I), D représente le polyéthylèneglycol, le polypropylèneglycol, la polylysine ou des dendrimères de polylysine ou leurs dérivés.

10. Produit diagnostique optique pour le diagnostic in vivo de zones de tissu malades au moyen de rayonnement IR proche, **caractérisé en ce qu'**il contient au moins un composé selon la revendication 1 avec les adjuvants et/ou supports usuels ainsi que des diluants.

11. Composés selon la revendication 1, **caractérisés en ce que** dans la formule générale (I), L représente une structure de lien dissociable par voie enzymatique, consistant en une séquence peptidique à courte chaîne.
